# EUROPEAN PATENT APPLICATION

(11) **EP 4 397 262 A1**
(43) Date of publication of application: **10.07.2024**
(21) Application number: 23213327.2
(22) Date of filing: 30.11.2023
(51) Int. Cl.: A61B 18/14, A61B 17/00

(54) **ELECTRODE FOR A HANDHELD ELECTROSURGICAL INSTRUMENT**

(30) Priority: 05.01.2023 US 202363437157 P
(71) Applicant: Olympus Winter & Ibe GmbH, 22045 Hamburg (DE)
(72) Inventor: Knopf, Christoph, 23617 Stockelsdorf (DE); Brockmann, Christian, 21279 Hollenstedt (DE); Lavicka, Jiri, 75002 Prerov (CZ); Komínek, Petr, 75002 Prerov (CZ)
(74) Representative: Hoener, Matthias

(57) **Abstract**

The invention creates an electrode which can be used particularly efficiently and can be manufactured in a particularly cost-effective manner. This is achieved in that an electrode (16) for an electrosurgical hand instrument consists of an electrically conductive wire (18), the two ends of the wire (18) being connectable to an electrode support (14) of the hand instrument. Thereby, the wire (18) has two sections R1 and L1, which are adjacent to the two ends of the wire (18) and are parallel to each other and aligned straight. These two first sections R2 and L2 are adjoined by two second sections R2 and L2, these two sections R2 and L2 being connected to each other by a section C. This section C in turn has a flattened section CC.

## Description

The invention relates to an electrode for an electrosurgical hand instrument according to claim 1.

Electrosurgical hand-held devices, in particular resectoscopes, of the type described are used primarily in urology for electrosurgical work. In this context, these devices are usually used for resection and for evaporation of tissue, for example of tissue in the lower urinary tract. For this purpose, the hand-held device, in particular the resectoscope, may comprise a longitudinally displaceable electrode carrier which, after insertion of the device into the body to be treated, may be advanced with a distal working end from a distal end of the instrument shaft of the hand-held device. An electrosurgical electrode is disposed on the electrode carrier at a distal end thereof. This electrode may be in the form of a loop, for example, and is pulled or pushed through the tissue to manipulate the tissue, depending on the design of the instrument.

Known electrodes are formed from a wire with a circular cross-section. For loop electrodes, for example, it is known that the wire with the circular cross-section is formed into a loop and the two free ends are connected to the electrode support. When the high-frequency alternating voltage is applied, a plasma ignites at the electrode, so that a plasma is formed at the wire in interaction with the liquid surrounding the electrode. The plasma ignites preferentially at positions of the electrode that have a small radius of curvature, since the electric field strength is greatest there. In the case of a continuous wire as electrode with a constant cross-section, the plasma ignites statistically distributed over the entire length of the electrode. A preferred position for plasma ignition can therefore not develop. However, this turns out to be disadvantageous for the manipulation of the body tissue, since the plasma ignites only rarely at a certain position of the electrode and thus not reliably, namely randomly. In addition, the contact area of the loop electrode with the tissue is small due to its circular cross-section, so that the coagulation performance is lower compared to other electrode shapes.

In another known embodiment of an electrode, the so-called strip electrode, it is provided that first a single loop with a rectangular cross-section is produced and this loop is then welded at its two ends with short wires, which in turn are to be coupled to the electrode support. Such electrodes are also particularly complex and cost-intensive to manufacture. In addition, the plasma also ignites in these strip electrodes not preferentially at one position, but statistically distributed over the entire length and thus also at less advantageous positions, such as at the transitions to the wires.

For the manufacture of known electrodes, tubes are therefore used which are compressed so that the cross-section is oval-shaped. Due to the different radii of curvature of the compressed tube, areas of different field strength are created along the tube, thus creating areas where the plasma preferentially ignites. However, this type of electrode is particularly cost-intensive to manufacture, since thin tubes or pipes made of materials such as a platinum-iridium alloy in particular are very expensive.

The invention is therefore based on the problem of creating an electrode that can be used particularly efficiently and can be manufactured in a particularly cost-effective manner.

A solution to this problem is described by claim 1. Accordingly, it is provided that an electrode for an electrosurgical hand instrument, in particular for a resectoscope, consists of an electrically conductive wire, the two ends of the wire being connectable to an electrode support of the hand instrument. In this case, the wire has two sections R1 and L1, which are adjacent to the two ends of the wire and are parallel to one another and aligned straight. These two first sections R1 and L1 are adjoined by two second sections R2 and L2, these two sections R2 and L2 being connected to each other by a section C. This section C in turn has a flattened section CC. This flattened section CC may be identical to the length of section C or may represent only a small section of C.

Preferably, the invention provides that the wire has a round or circular cross-section and the flattened section CC has an oval or elliptical cross-section or a rectangular cross-section with rounded corners. The cross-section of section CC thus differs from the cross-sections of the other wire sections in that it has different radii of curvature. Thereby, cross-section of section C is formed in such a way that it has a smallest radius of curvature compared to all other sections of the wire. This smallest radius of curvature is limited to section C or section CC. Since the plasma ignites preferentially and particularly quickly or breaks through the gas phase where the electric field strength is greatest, the probability of plasma ignition is greatest here. Since this represents only a limited section of the wire, the electrode can be used in a particularly targeted and reliable manner to manipulate the tissue. A further advantage of the feature is that the electrode is made of a wire which, on the one hand, is very inexpensive to purchase and, on the other hand, is particularly easy to shape or handle.

In particular, the invention may provide that sections R1 and L1 and sections R2 and L2 have a round or circular cross-section. If the section CC occupies only part of the section C, the non-flattened part of the section C also has a round or circular cross-section. This shape of the remaining sections, where the electric field strength is rather low due to the constant shape of the cross-section, can additionally promote that the plasma in section C or CC ignites very quickly and reliably.

It is further conceivable that section C has a radius of 2.8 mm to 3.5 mm or is straight or is aligned at a right angle to sections R2 and L2 or has a V-shape. Depending on the type of electrode or area of application, section C can be formed differently. In the case of a loop electrode, however, the section C or the section CC is formed in the manner of a circular section and can, for example, include an angle ω of 110° to 160° between the two sections R2 and L2. In this case, only the section CC is flattened with the mentioned radius. In the transition from section CC to section C or from section C to sections R2 and L2, the cross-sectional shape of the wire continuously changes from the flattened shape to the circular shape.

In addition, it can be provided according to the invention that sections R2 and L2 also have a radius or are aligned parallel to one another and/or that the transitions between sections L1 and L2, R1 and R2 and between C and L2 and R2 have a radius. It is conceivable that the radius of the sections R2 and L2 is also in the range of 2.8 mm to 3.5 mm or is different from the radius of the section C. By means of a radius of the sections R2 and L2, the transition of the different cross-sectional shapes from the sections R2 and L2 to the section C can be made smoother, if necessary, so that no radii of curvature are formed at these transition points which are smaller than the smallest radius of curvature of the section C. Similarly, it is also conceivable that the two sections R2 and L2 are parallel to each other and aligned straight and, on the one hand, preferably lie in the same plane as the sections R1 and L1 and, on the other hand, lie in the same plane as the section C.

A particularly advantageous embodiment of the invention may provide that the wire has a diameter of 0.4 mm to 0.6 mm, preferably 0.5 mm. The cross-section of the flattened section CC has a height-to-width ratio of 1:1.5 to 1:4. Preferably, this ratio is 1:2. In a particularly preferred embodiment, a height of the flattened section CC is 0.3 mm and a width is 0.6 mm. It should be explicitly noted that the corners of the flattened cross-section or the rectangular cross-section are round, so that a rectangle is not formed, but rather a cross-section with a continuous contour. Due to this shape, a plasma is formed particularly preferentially around section CC or at the areas of section CC with the smallest radius of curvature.

It is known that material erosion occurs in the course of the application of the electrode, which can lead to a change in the shape of the electrode. This can be avoided by the shape of the section CC described here. Due to the rounded shape of the flattened section CC, material erosion is distributed over the entire length or circumference of the wire, so that the electrode can be used with the necessary reliability over a longer period of time.

Another preferred embodiment of the invention may provide that the wire is formed of tungsten, stainless steel, platinum-iridium, titanium, a titanium alloy or a platinum-tungsten alloy. These materials are particularly advantageous because they are electrically conductive, have high electrical as well as mechanical stability and high resistance to plasma erosion, and are easily machinable or deformable mechanically.

It may further be provided in accordance with the invention that the lengths of the sections R2 and L2 are 0.7 mm to 1.7 mm, and that the two second sections R2 and L2 enclose an angle α of 35° to 120°, preferably 90°, 45° or 110°, with the first sections R1 and L1. It may be further provided that the sections R2 and L2 and C and CC lie in a common plane. It has been shown that this dimensioning and these relative arrangements are particularly advantageous for efficient treatment of the patient and for particularly cost-effective manufacture of the electrode. Depending on the type of application of the electrode, different angles can be chosen between the two pairs of sections. Sections R1 and R2 and L1 and L2 each lie in a plane, these planes being aligned parallel to one another. By shaping the various sections in this way according to the invention, a large number of different electrode shapes can be produced in a particularly simple and thus inexpensive manner. Furthermore, another embodiment of the invention may provide that bending radii between sections R1 and R2 and L1 and L2 are 0.1 mm to 1 mm.

A preferred embodiment of the invention is described in more detail below with reference to the drawing. In this show:
- Fig. 1: a schematic representation of a handheld surgical device, in particular a resectoscope,
- Fig. 2: a side view of an electrode, and
- Fig. 3: a front view of the electrode.

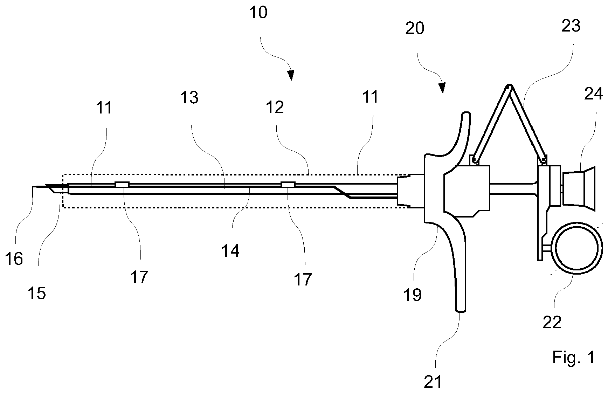

Fig. 1 shows a schematic, side cross-sectional view of a resectoscope known as 10. The resectoscope 10 has a resectoscope shaft 11 that includes an outer shaft 12 or sheath tube. A tubular inner shaft 13 extends within the outer shaft 12. A electrode array 14 and an indicated optic 15 are shown within the inner shaft 13. In addition, other elements not shown here may be disposed within the resectoscope 10, such as a separate irrigation tube and the like.

The electrode array 14 has an electrosurgical tool or electrode 16 at a distal end. The electrode 16 shown here is shown as a loop, but may also be formed as a button or the like.

The electrode holder 14 can be forcibly moved axially in the distal and proximal direction by actuating a handle 19. In doing so, it can be pushed beyond the distal end of the inner shaft 13 and the outer shaft 12. This allows the surgeon to manipulate tissue further away from the resectoscope tip. Furthermore, for this purpose, the inner shaft 13 and/or the electrode carrier 14 can be rotatably mounted about their longitudinal axis. For the manipulation of the tissue, a high-frequency electric current is applied to the electrode 16.

The resectoscope 10 shown in Fig. 1 has a passive transporter in which a carriage 20 is displaced in the distal direction against the distal, first handle part 21 by relative movement of the handle parts 21 and 22 arranged proximally on the resectoscope shaft 11 against a spring force applied by a spring bridge 23. When the carriage 20 is displaced in the distal direction against the handle part 21, the electrode support 14 is displaced in the distal direction in a manner not shown. When the load on the handle parts 21, 22 is relieved, the spring force generated by the spring bridge 23 forces the slide 20 back to its initial position, pulling the electrode carrier 14 in the proximal direction. When the slide 20 is moved back, an electrosurgical procedure can be performed with the electrode 16 without any manual force from the operator, i.e. passively.

For targeted treatment by means of the electrode 16, the optics 15 are positioned in such a way that the surgeon has an optimal view of the area of the operation. For this purpose, the resectoscope 10 has an eyepiece 24 at a proximal end, which is connected to the optics 15. Alternatively, it is also conceivable that a camera is arranged on the resectoscope 10 instead of the eyepiece 24.

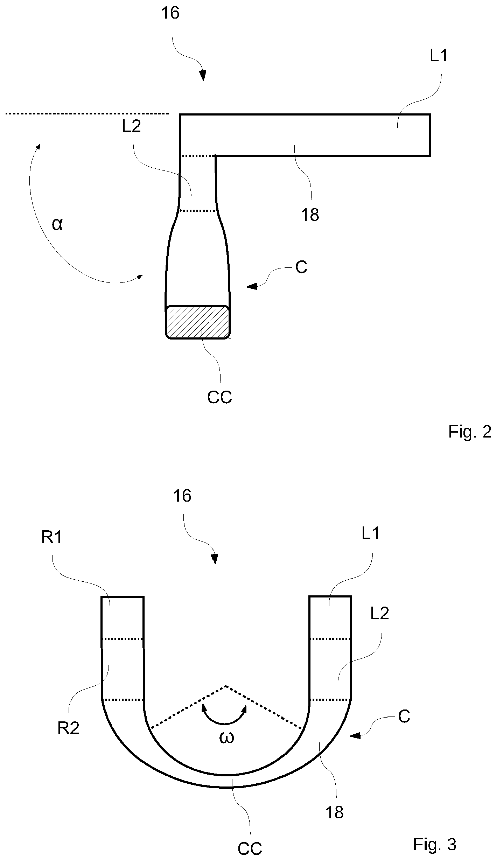

Figs. 2 and 3 show an embodiment example of an electrode 16 according to the invention. This electrode 19 has first sections R1 and L1, wherein the first sections R1 and L1 are equally pronounced in length and oriented parallel to each other. These first sections R1 and L1 are connected to the distal ends of the electrode support tubes. This connection or coupling to the electrode support tubes stabilizes the electrode 16 as well as applies electrical energy to it. The first sections R1 and L1 are followed by the two second sections R2 and L2. These two second sections R2 and L2 are also formed with the same length and are aligned parallel to each other. The transition from the first sections R1 and L1 to the second sections L2 and L2 takes place in one plane. In the embodiment example of the electrode 16 shown here, the second sections R2 and L2 are inclined at an angle of 90° with respect to the first sections R1 and L1. However, it is also conceivable that this angle α assumes a value between 35° and 120°, preferably 45°, 90° or 110°, or any other angle.

Section C is located between the two second sections R2 and L2. This section C connects the two second sections R2 and L2 and is formed as a loop in the embodiment examples shown. The shape of the section C may also vary and may, for example, have a larger or smaller radius of curvature. In the present embodiment example, the section C lies in the same plane as the sections R2 and L2. To perform the operation, the electrode 16 is pulled or pushed through the tissue to be treated with the section C, depending on the type of resectoscope 10.

According to the invention, the electrode 16 consists of a wire 18, preferably a wire 18 made of tungsten, stainless steel, platinum-iridium, titanium, a titanium alloy or a platinum-tungsten alloy. This wire 18 and, in particular, the sections L1 and L2 as well as R1 and R2 and, if necessary, also in sections the section C have a round or circular cross-section (Figs. 2, 3). According to the invention, the wire 18 in section C has a flattened section CC. In this section CC, the otherwise circular wire 18 is figuratively flattened so that the cross-section is not circular, but rather describes oval, elliptical or a rectangle with rounded corners. This cross-sectional shape of a rectangle with rounded corners is sketchily shown in Fig. 2. As a result, the height-to-width ratio of the cross-section of the section CC is 1:1.5 to 1:4 and, in particular, 1:2. Specifically, it is conceivable that the height of the cross-section of the section C is 0.3 mm and the width is 0.6 mm, the diameter of the wire 18 being 0.4 mm to 0.6 mm, preferably 0.5 mm. This results in the wire 18 in section CC being wider than sections L1, L2, R1 and R2 in the side view according to Fig. 2 and narrower in the front view according to Fig. 3. This deformation of the section CC causes the electric field strength to be particularly high, especially at the rounded corners, which results in the plasma generated by the high-frequency AC voltage igniting at least almost exclusively at these rounded corners of the section CC. That is, the plasma is generated locally in a very defined and reliable manner, thus enabling reliable and targeted treatment of the tissue. Furthermore, the flattened shape of the section CC proves to be particularly suitable for manipulating tissue in the application of the resectoscope 10 described above. Furthermore, an advantage can be seen in the fact that the wear of the electrode caused by the plasma is distributed over the rounded corners of the section CC and thus does not exert any further effects on the functionality of the electrode 16.

Another embodiment of the invention may provide that not only the section C has a radius, i.e., a loop shape, but also the adjacent sections R2 and L2 have a radius and are oriented toward each other to accommodate the loop-like shape of the section C. This slight angling of sections R2 and L2 allows any further radii of curvature of electrode 16 to be minimized in order to concentrate the plasma on section CC.

Another possible embodiment of the invention may provide that the flattened area of the section C, i.e. the section CC, is limited to an angular range ω of 110° to 160° between the sections R1 and L1 (in the section C). Specifically, it is conceivable that only a very narrow area of section C is flattened. This appears to be advantageous for special applications, for example. Thus, it is conceivable that a set of different electrodes 16 is available to the surgeon with flattened sections CC of different widths, which can be selected according to the application and can be coupled to the resectoscope 10.

### List of Reference Numerals:

- 10: Resectoscope
- 11: Resectoscope shaft
- 12: Outer shaft
- 13: Inner shaft
- 14: Electrode carrier
- 15: Optics
- 16: Electrode
- 17: Guide element
- 18: Wire
- 19: Handle
- 20: Sled
- 21: Handle part
- 22: Handle part
- 23: Spring bridge
- 24: Ocular

- R1: First section
- R2: Second section
- L1: First section
- L2: Second section
- C: Central section
- CC: Flattened section

- α: Angle
- ω: Angle

## Claims

1. Electrode (16) for an electrosurgical hand instrument, in particular for a resectoscope (10), consisting of an electrically conductive wire (18), wherein the two ends of the wire (18) can be connected to an electrode carrier (14) of the hand instrument, **characterised in that** two sections R1 and L1 of the wire (18), which adjoin the two ends of the wire (18) are aligned parallel to one another and straight, and two second sections R2 and L2 adjoin the first sections R1 and L1, and the two sections R2 and L2 being connected to one another by a section C, and the wire (18) having a flattened section CC in the section C.

2. Electrode (16) for an electrosurgical hand instrument according to claim 1, **characterized in that** the wire (18) has a round or circular cross-section and the flattened portion CC of C has an oval or elliptical cross-section or a rectangular cross-section with rounded corners.

3. Electrode (16) for an electrosurgical hand instrument according to claim 1 or 2, **characterized in that** the sections R1 and L1 and the sections R2 and L2 have a round and circular cross-section, respectively.

4. Electrode (16) for an electrosurgical hand instrument according to any of the preceding claims, **characterized in that** the section C has a radius of 2.8 mm to 3.5 mm or is straight or is oriented at a right angle to the sections R2 and L2 or has a V-shape.

5. Electrode (16) for an electrosurgical hand instrument according to any one of the preceding claims, **characterized in that** the sections R2 and L2 have a radius or are aligned parallel to each other and/or that the transitions between the sections L1 and L2, R1 and R2 and between C and L2 and R2 have a radius.

6. Electrode (16) for an electrosurgical hand instrument according to any of the preceding claims, **characterized in that** the wire (18) has a diameter of 0.4 mm to 0.6 mm, preferably 0.5 mm.

7. Electrode (16) for an electrosurgical hand instrument according to any one of the preceding claims, **characterized in that** the cross-section of the flattened section CC has a height-to-width ratio of 1:1.5 to 1:4, preferably 1:2.

8. Electrode (16) for an electrosurgical hand instrument according to any one of the preceding claims, **characterized in that** the cross-section of the flattened portion CC has a height of 0.3 mm and a width of 0.6 mm.

9. Electrode (16) for an electrosurgical hand instrument according to any one of the preceding claims, **characterized in that** the flattened portion CC includes an angle ω of 110° to 160°.

10. Electrode (16) for an electrosurgical hand instrument according to any one of the preceding claims, **characterized in that** the wire (18) is made of tungsten, stainless steel, platinum-iridium, titanium, a titanium alloy or a platinum-tungsten alloy.

11. Electrode (16) for an electrosurgical hand instrument according to any of the preceding claims, **characterized in that** the lengths of the sections R2 and L2 are 0.7 mm to 1.7 mm.

12. Electrode (16) for an electrosurgical hand instrument according to any one of the preceding claims, **characterized in that** the two second sections R2 and L2 enclose with the first sections R1 and L1 an angle α of 35° to 120°, preferably 90°, 45° or 110°.

13. Electrode (16) for an electrosurgical hand instrument according to any of the preceding claims, **characterized in that** the sections R2, L2 and C and CC lie in one plane.
